# EUROPEAN PATENT SPECIFICATION

(11) **EP 4 218 588 B1**
(45) Date of publication and mention of the grant of the patent: **14.05.2025**
(21) Application number: 21872445.8
(22) Date of filing: 21.09.2021
(51) Int. Cl.: A61B 6/00

(54) **CONTROL DEVICE, CONTROL METHOD, AND CONTROL PROGRAM**
STEUERUNGSVORRICHTUNG, STEUERUNGSVERFAHREN UND STEUERUNGSPROGRAMM
DISPOSITIF DE COMMANDE, PROCÉDÉ DE COMMANDE ET PROGRAMME DE COMMANDE

(30) Priority: 28.09.2020 JP 2020162697
(43) Date of publication of application: 02.08.2023
(73) Proprietor: FUJIFILM Corporation, Tokyo 106-8620 (JP)
(72) Inventor: FUKUDA, Wataru, Ashigarakami-gun, Kanagawa 258-8538 (JP)
(74) Representative: Dehns Germany Partnerschaft mbB
(86) International application number: PCT/JP2021/034624
(87) International publication number: WO 2022/065316

(56) References cited:
- EP-A1- 3 643 222
- WO-A1-2007/049471
- WO-A1-2015/107963
- JP-A- 2004 248 932
- US-A1- 2005 084 073
- US-A1- 2019 370 956

## Description

### Technical Field

The present disclosure relates to a control apparatus, a control method, and a non-transitory storage medium storing a control program.

### Related Art

A technology of performing contrast imaging of capturing a low-energy image and a high-energy image by irradiating a subject into which a contrast medium has been injected with radiation having different energies and generating a difference image showing a difference between the high-energy image and the low-energy image to generate a radiation image in which the contrast medium is enhanced is known. It is desired to perform the contrast imaging in a state in which the contrast medium has sufficiently permeated the subject, and it is desired to capture at least the high-energy image at an appropriate timing. Therefore, a technology of controlling a timing of performing the contrast imaging is known. For example, JP2000-175900A discloses a technology of starting an examination scan of an examinee based on a change in a computed tomography (CT) value of a region of interest in a pre-scan after injecting a contrast medium. The present invention is characterised over US2005/084073, which discloses an apparatus for use in a radiation procedure that can generate volumetric composite images for different elapsed times after injection of a contrast agent, where for each time a volumetric composite image can be constructed from volumetric images associated with first and second radiation energy levels. US 2019/370956 A1 discloses a contrast imaging system.

### SUMMARY OF INVENTION

### Technical Problem

By the way, the ease of permeation of the contrast medium, that is, a speed at which the contrast medium permeates, or the like may differ according to the subject or the like. Therefore, it is difficult to specify an appropriate imaging timing, and even the technology disclosed in JP2000-175900A may not be sufficient in some cases.

### Solution to Problem

The present disclosure is made in view of the above circumstances, and provides a control apparatus, a control method, and a non-transitory storage medium storing a control program capable of capturing a high-energy image at an appropriate timing in contrast imaging.

A first aspect of the present disclosure relates to a control apparatus comprising at least one processor, in which the processor acquires an elapsed time from injection of a contrast medium into a subject for which a radiation image is captured by a radiography apparatus, acquires a low-energy image captured by the radiography apparatus by emitting radiation having first energy to the subject into which the contrast medium has been injected, and a high-energy image captured by the radiography apparatus by emitting radiation having second energy higher than the first energy to the subject into which the contrast medium has been injected, generates a difference image showing a difference between the low-energy image and the high-energy image, and identifies whether or not to perform re-capturing of the high-energy image of the subject into which the contrast medium has been injected, based on an analysis result about a contrast amount, which is performed on the difference image, and the elapsed time.

A second aspect of the present disclosure relates to the control apparatus according to the first aspect, in which, in a case in which the processor identifies that the re-capturing is to be performed, the processor derives a timing for performing the re-capturing based on the analysis result and the elapsed time.

A third aspect of the present disclosure relates to the control apparatus according to the second aspect, in which the processor performs control of causing the radiography apparatus to re-capture the high-energy image at the derived timing for performing the re-capturing.

A fourth aspect of the present disclosure relates to the control apparatus according to any one of the first to third aspects, in which, in a case in which a contrast of a region of interest in the difference image is equal to or greater than a predetermined threshold value, the processor identifies that the re-capturing is not to be performed regardless of the elapsed time.

A fifth aspect of the present disclosure relates to the control apparatus according to any one of the first to fourth aspects, in which, in a case in which a contrast of a region of interest in the difference image is smaller than a predetermined threshold value and the elapsed time is equal to or longer than a threshold value time, the processor identifies that the re-capturing is not to be performed.

A sixth aspect of the present disclosure relates to the control apparatus according to any one of the first to fourth aspects, in which, in a case in which a contrast of a region of interest in the difference image is smaller than a predetermined threshold value and the elapsed time is shorter than a threshold value time, the processor identifies that the re-capturing is to be performed.

A seventh aspect of the present disclosure relates to the control apparatus according to the fifth or sixth aspect, in which the threshold value time is a time according to at least one of a type of the contrast medium, a blood flow of the subject, a height of the subject, a weight of the subject, or a type of an object of interest.

An eighth aspect of the present disclosure relates to the control apparatus according to the fifth or sixth aspect, in which the subject is a breast, and the threshold value time is a time according to a mammary gland mass of the breast.

A ninth aspect of the present disclosure relates to the control apparatus according to any one of the first to eighth aspects, in which, in a case in which the processor identifies that the re-capturing is to be performed, the processor acquires the high-energy image re-captured by the radiography apparatus, and generates a re-captured difference image showing a difference between the low-energy image and the re-captured high-energy image.

A tenth aspect of the present disclosure relates to the control apparatus according to any one of the first to eighth aspects, in which, in a case in which the processor identifies that the re-capturing is to be performed, the processor also specifies that re-capturing of the low-energy image is to be performed, and generates a re-captured difference image showing a difference between the re-captured low-energy image and the re-captured high-energy image.

An eleventh aspect of the present disclosure relates to the control apparatus according to the ninth or tenth aspect, in which the processor performs the same image processing of enhancing a region of interest on the difference image and the re-captured difference image, and displays the difference image and the re-captured difference image which have been subjected to the image processing.

A twelfth aspect of the present disclosure relates to the control apparatus according to any one of the ninth to eleventh aspects, in which the processor further displays contrast amount information about a contrast amount of the same region of each of the difference image and the re-captured difference image.

A thirteenth aspect of the present disclosure relates to the control apparatus according to any one of the first to twelfth aspects, in which the subject is a breast, and the radiography apparatus is a mammography apparatus.

In addition, a fourteenth aspect of the present disclosure relates to a control method executed by a computer, the method comprising acquiring an elapsed time from injection of a contrast medium into a subject for which a radiation image is captured by a radiography apparatus, acquiring a low-energy image captured by the radiography apparatus by emitting radiation having first energy to the subject into which the contrast medium has been injected, and a high-energy image captured by the radiography apparatus by emitting radiation having second energy higher than the first energy to the subject into which the contrast medium has been injected, generating a difference image showing a difference between the low-energy image and the high-energy image, and identifying whether or not to perform re-capturing of the high-energy image of the subject into which the contrast medium has been injected, based on an analysis result about a contrast amount, which is performed on the difference image, and the elapsed time.

In addition, a fifteenth aspect of the present disclosure relates to a non-transitory storage medium storing a program causing a computer to execute a control processing, the control processing comprising: acquiring an elapsed time from injection of a contrast medium into a subject for which a radiation image is captured by a radiography apparatus, acquiring a low-energy image captured by the radiography apparatus by emitting radiation having first energy to the subject into which the contrast medium has been injected, and a high-energy image captured by the radiography apparatus by emitting radiation having second energy higher than the first energy to the subject into which the contrast medium has been injected, generating a difference image showing a difference between the low-energy image and the high-energy image, and identifying whether or not to perform re-capturing of the high-energy image of the subject into which the contrast medium has been injected, based on an analysis result about a contrast amount, which is performed on the difference image, and the elapsed time.

### Effects of Invention

According to the present disclosure, it is possible to capture the high-energy image at an appropriate timing in the contrast imaging. The invention is defined in the appended set of claims.

### BRIEF DESCRIPTION OF THE DRAWINGS

Fig. 1 is a configuration diagram schematically showing an example of an overall configuration of a radiography system according to an embodiment.
Fig. 2 is a side view showing an example of an appearance of a mammography apparatus according to the embodiment.
Fig. 3 is a block diagram showing an example of a configuration of a console according to the embodiment.
Fig. 4 is a functional block diagram showing an example of a function of the console according to the embodiment.
Fig. 5 is a flowchart showing an example of a flow of contrast imaging by the radiography system according to the embodiment.
Fig. 6A is a time chart showing an example of the contrast imaging in a case in which re-capturing is performed.
Fig. 6B is a time chart showing an example of the contrast imaging in a case in which the re-capturing is not performed.
Fig. 7 is a flowchart showing an example of a flow of contrast imaging control processing executed in the contrast imaging.
Fig. 8 is a flowchart showing an example of a flow of difference image display processing executed in the contrast imaging.
Fig. 9 is a diagram showing an example of a state in which a difference image and contrast amount information are displayed on a display unit.
Fig. 10 is a time chart showing an example of a case in which the re-capturing of only a high-energy image is performed.

### DESCRIPTION OF EMBODIMENTS

In the following, an embodiment of the present invention will be described in detail with reference to the drawings. It should be noted that the present embodiment does not limit the present invention.

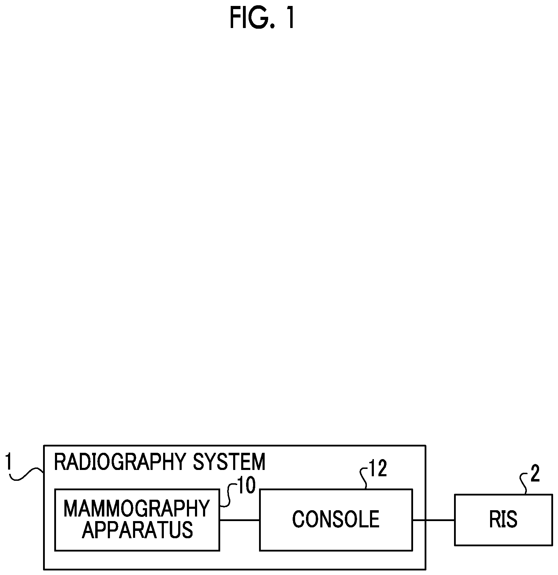

First, an example of an overall configuration of a radiography system according to the present embodiment will be described. Fig. 1 shows a configuration diagram showing an example of an overall configuration of a radiography system 1 according to the present embodiment. As shown in Fig. 1, the radiography system 1 according to the present embodiment comprises a mammography apparatus 10 and a console 12. The mammography apparatus 10 according to the present embodiment is an example of a radiography apparatus according to the present disclosure. In addition, the console 12 according to the present embodiment is an example of a control apparatus according to the present disclosure.

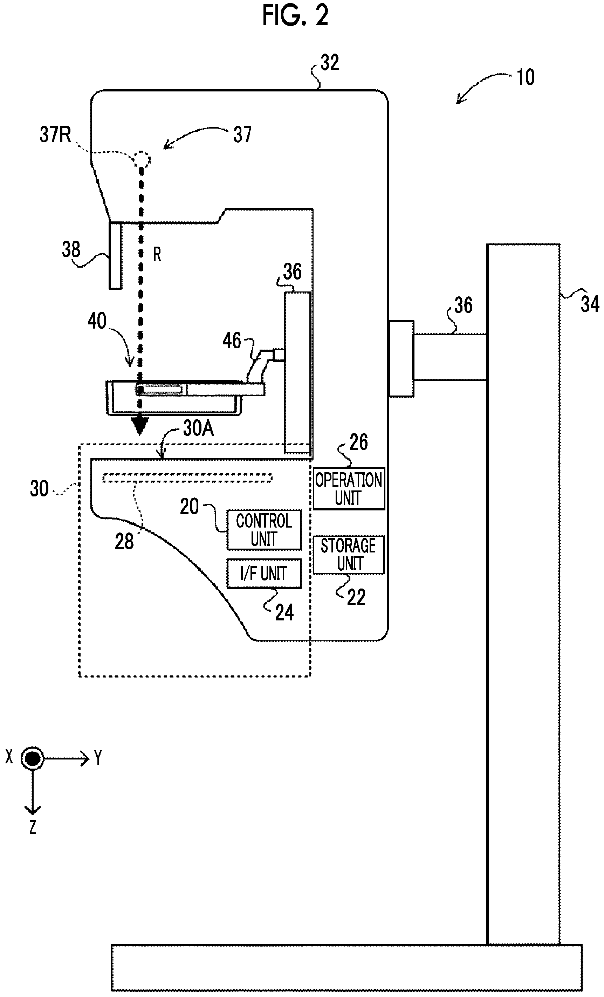

First, the mammography apparatus 10 according to the present embodiment will be described. Fig. 2 shows a side view showing an example of an appearance of the mammography apparatus 10 according to the present embodiment. It should be noted that Fig. 2 shows the example of the appearance of the mammography apparatus 10 as viewed from a right side of an examinee.

The mammography apparatus 10 according to the present embodiment is an apparatus that uses a breast of the examinee as a subject and captures a radiation image of the breast by irradiating the breast with radiation R (for example, X-rays). It should be noted that the mammography apparatus 10 may be an apparatus that images the breast of the examinee in a state in which the examinee is sitting on a chair (including a wheelchair) or the like (sitting state) in addition to a state in which the examinee is standing (standing state).

In addition, the mammography apparatus 10 according to the present embodiment has a function of performing two types of imaging of so-called contrast imaging in which the imaging is performed in a state in which a contrast medium has been injected into the breast of the examinee and general imaging. It should be noted that, in the present embodiment, the imaging to be performed in a state in which the contrast medium has been injected into the breast of the examinee refers to the "contrast imaging", and the imaging that is not the contrast imaging refers to the "general imaging".

As shown in Fig. 2, the mammography apparatus 10 according to the present embodiment comprises a control unit 20, a storage unit 22, an interface (I/F) unit 24, and an operation unit 26 inside the imaging table 30. The control unit 20 controls an overall operation of the mammography apparatus 10 under the control of the console 12. The control unit 20 comprises a central processing unit (CPU), a read only memory (ROM), and a random access memory (RAM) (all not shown). The ROM stores, in advance, various programs, including an imaging processing program for performing control related to radiation image capturing, which is executed by the CPU. The RAM transitorily stores various data.

The storage unit 22 stores the image data of the radiation image captured by the radiation detector 28 or various types of other information. Specific examples of the storage unit 22 include a hard disk drive (HDD) and a solid state drive (SSD). The I/F unit 24 performs communication of various types of information with the console 12 by wireless communication or wired communication. The image data of the radiation image captured by the radiation detector 28 in the mammography apparatus 10 is transmitted to the console 12 via the I/F unit 24 by wireless communication or wired communication.

In addition, an operation unit 26 is provided as a plurality of switches on an imaging table 30 of the mammography apparatus 10, for example. It should be noted that the operation unit 26 may be provided as a touch panel type switch, or may be provided as a foot switch operated by a user, such as a doctor or an engineer with a foot.

The radiation detector 28 detects the radiation R that has passed through the breast which is the subject. In addition, as shown in Fig. 2, the radiation detector 28 is disposed inside the imaging table 30. In the mammography apparatus 10 according to the present embodiment, the user positions the breast of the examinee on an imaging surface 30A of the imaging table 30 in a case of performing the imaging.

The radiation detector 28 detects the radiation R transmitted through the breast of the examinee and the imaging table 30, generates a radiation image based on the detected radiation R, and outputs image data representing the generated radiation image. A type of the radiation detector 28 according to the present embodiment is not particularly limited. For example, a radiation detector of an indirect conversion method that converts the radiation R into light and converts the converted light into a charge may be used, and a radiation detector of a direct conversion method that directly converts the radiation R into a charge may be used.

A radiation emitting unit 37 comprises the radiation source 37R. As shown in Fig. 2, the radiation emitting unit 37 is provided in an arm part 32 together with the imaging table 30 and the compression unit 36. As shown in Fig. 2, a face guard 38 is attachably and detachably provided at a position near the examinee on the arm part 32 below the radiation emitting unit 37. The face guard 38 is a protective member for protecting the examinee from the radiation R emitted from the radiation source 37R.

It should be noted that, as shown in Fig. 2, the mammography apparatus 10 according to the present embodiment comprises the arm part 32, a base 34, and a shaft part 35. The arm part 32 is held by the base 34 to be movable in a vertical direction (Z-axis direction). The shaft part 35 connects the arm part 32 to the base 34. In addition, the arm part 32 is rotatable relative to the base 34 with the shaft part 35 as a rotation axis.

The arm part 32, the imaging table 30, and the compression unit 36 can be separately rotated relative to the base 34 with the shaft part 35 as a rotation axis. In the present embodiment, the base 34, the arm part 32, the imaging table 30, and the compression unit 36 are each provided with an engaging part (not shown), and each of the arm part 32, the imaging table 30, and the compression unit 36 is connected to the base 34 by switching a state of the engaging part. One or two of the arm part 32, the imaging table 30, or the compression unit 36, which are connected to the shaft part 35, are integrally rotated around the shaft part 35.

The compression unit 36 is provided with a compression plate driving unit (not shown) that moves the compression plate 40 in the vertical direction (Z-axis direction). The compression plate 40 according to the present embodiment has a function of compressing the breast of the examinee. A support part 46 of the compression plate 40 is attachably and detachably attached to the compression plate driving unit, is moved in the vertical direction (Z-axis direction) by the compression plate driving unit, and compresses the breast of the examinee with the imaging table 30.

On the other hand, the console 12 according to the present embodiment has a function of controlling the mammography apparatus 10 by using an imaging order and various types of information acquired from a radiology information system (RIS) 2 via a wireless communication local area network (LAN) and the like, and an instruction performed by the user by an operation unit 56 and the like.

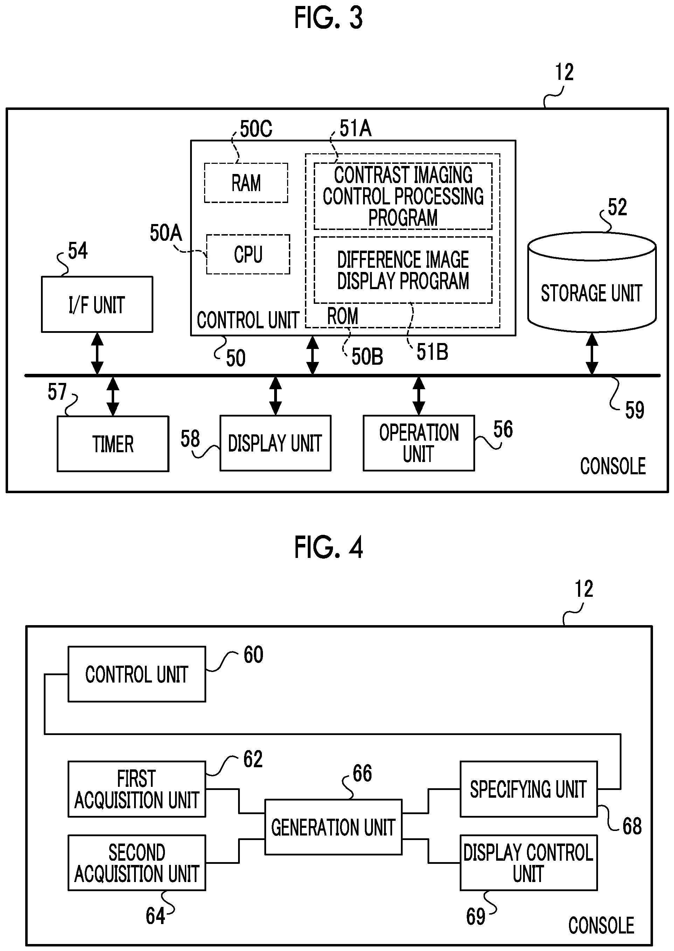

The console 12 according to the present embodiment is, for example, a server computer. As shown in Fig. 3, the console 12 comprises a control unit 50, a storage unit 52, an I/F unit 54, the operation unit 56, a timer 57, and a display unit 58. The control unit 50, the storage unit 52, the I/F unit 54, the operation unit 56, the timer 57, and the display unit 58 are connected to each other via a bus 59, such as a system bus or a control bus, such that various types of information can be exchanged.

The control unit 50 according to the present embodiment controls an overall operation of the console 12. The control unit 50 comprises a CPU 50A, a ROM 50B, and a RAM 50C. The ROM 50B stores, in advance, various programs including a contrast imaging control processing program 51A and a difference image display program 51B, which are executed by the CPU 50A and will be described below. The RAM 50C transitorily stores various data. The CPU 50A according to the present embodiment is an example of a processor according to the present disclosure. The contrast imaging control processing program 51A according to the present embodiment is an example of a control program according to the present disclosure.

The storage unit 52 stores the image data of the radiation image captured by the mammography apparatus 10 or various types of other information. Specific examples of the storage unit 52 include an HDD and an SSD.

The operation unit 56 is used by the user to input the instruction, various types of information, and the like related to the radiation image capturing and the like, including an irradiation instruction of the radiation R. The operation unit 56 is not particularly limited, and examples thereof include various switches, a touch panel, a touch pen, and a mouse. The display unit 58 displays various types of information. It should be noted that the operation unit 56 and the display unit 58 may be integrated to form a touch panel display.

The I/F unit 54 performs communication of various types of information between the mammography apparatus 10 and the RIS 2 by wireless communication or wired communication. The console 12 according to the present embodiment receives the image data of the radiation image captured by the mammography apparatus 10 from the mammography apparatus 10 via the I/F unit 54 by wireless communication or wired communication.

The timer 57 starts counting in a case in which a start instruction is received from the user via the operation unit 56, and stops counting in a case in which a stop instruction is received from the user via the operation unit 56. Stated another way, the timer 57 counts the time from the reception of the start instruction by the user to the reception of the stop instruction.

Further, Fig. 4 shows a functional block diagram of an example of the configuration of the console 12 according to the present embodiment. As shown in Fig. 4, the console 12 comprises a control unit 60, a first acquisition unit 62, a second acquisition unit 64, a generation unit 66, and a specifying unit 68. As an example, in the console 12 according to the present embodiment, the CPU 50A of the control unit 50 also functions as the control unit 60, the first acquisition unit 62, the second acquisition unit 64, the generation unit 66, and the specifying unit 68 by the CPU 50A executing the contrast imaging control processing program 51A stored in the ROM 50B.

The control unit 60 has a function of performing control related to the irradiation with the radiation R in the mammography apparatus 10 in the contrast imaging. In the present embodiment, in a case of performing the contrast imaging, the radiation image is captured by emitting the radiation R having the first energy from the radiation source 37R to the breast in a state in which the contrast medium has been injected. In addition, the radiation image is captured by emitting the radiation R having the second energy higher than the first energy from the radiation source 37R to the breast in a state in which the contrast medium has been injected. It should be noted that, in the present embodiment, the radiation image captured by emitting the radiation R having the first energy is referred to as a "low-energy image", and the radiation image captured by emitting the radiation R having the second energy is referred to as a "high-energy image". In addition, in a case in which the images captured by the mammography apparatus 10 are collectively referred to without distinction between types, such as the low-energy image and the high-energy image, the images are simply referred to as the "radiation image".

For example, an iodine contrast medium with a k-absorption edge of 32 keV is generally used as the contrast medium for the contrast imaging. In the contrast imaging in this case, the low-energy image is captured by emitting the radiation R having the first energy lower than the k-absorption edge of the iodine contrast medium. In addition, the high-energy image is captured by emitting the radiation R having the second energy higher than the k-absorption edge of the iodine contrast medium.

Therefore, in the contrast imaging, the control unit 60 according to the present embodiment performs control of emitting the radiation R having the first energy from the radiation source 37R and control of emitting the radiation R having the second energy. **In** other words, the control unit 60 performs control of causing the mammography apparatus 10 to capture the low-energy image and control of causing the mammography apparatus 10 to capture the high-energy image.

A body tissue, such as a mammary gland, and the contrast medium have different absorption characteristics of the radiation. Therefore, in the high-energy image captured as described above, the body tissue, such as the mammary gland or fat, is reflected, and the contrast medium is clearly reflected. **In** addition, in the low-energy image, almost no contrast medium is reflected, and the body tissue, such as the mammary gland, is clearly reflected. Therefore, the difference image showing a difference between the low-energy image and the high-energy image can be made to be an image in which a mammary gland structure is removed and the contrast medium is clearly reflected.

In addition, the control unit 60 according to the present embodiment has a function of performing control of causing the mammography apparatus 10 to re-capture each of the low-energy image and the high-energy image in a case in which the specifying unit 68, which will be described in detail below, specifies that the re-capturing is to be performed.

The first acquisition unit 62 has a function of acquiring an elapsed time from the injection of the contrast medium into the breast. In the present embodiment, in a case in which the contrast medium is injected into the breast, the user operates the operation unit 56 to instruct the start of counting. According to the present instruction, the timer 57 starts counting, as described above. The first acquisition unit 62 acquires the time counted by the timer 57 as the elapsed time from the injection of the contrast medium into the breast (hereinafter, simply referred to as "elapsed time"). The first acquisition unit 62 outputs the acquired elapsed time to the generation unit 66.

The second acquisition unit 64 has a function of acquiring the low-energy image and the high-energy image captured by the mammography apparatus 10. Specifically, the acquisition unit 64 acquires image data representing the low-energy image and image data representing the high-energy image captured by the radiation detector 28 of the mammography apparatus 10 via the I/F unit 24 and the I/F unit 54. The second acquisition unit 64 outputs the acquired low-energy image and high-energy image to the generation unit 66.

The generation unit 66 has a function of generating a difference image showing a difference between the low-energy image and the high-energy image. As an example, the generation unit 66 according to the present embodiment generates the difference image data representing the difference image in which the mammary gland tissue is removed and the contrast medium is enhanced, by subtracting image data obtained by multiplying the low-energy image by a predetermined coefficient from image data obtained by multiplying the high-energy image by a predetermined coefficient for each corresponding pixel. It should be noted that, in a case in which the high-energy image is re-captured, the difference image based on the re-captured high-energy image is also generated. The difference image in this case is an example of the re-captured difference image according to the present disclosure. The generation unit 66 outputs the generated difference image and the elapsed time acquired from the first acquisition unit 62 in association with each other to each of the specifying unit 68 and the display control unit 69.

The specifying unit 68 has a function of performing an image analysis on the difference image as an analysis about a contrast amount, and specifying whether or not to perform the re-capturing of the low-energy image and the high-energy image of the breast into which the contrast medium has been injected, based on the analysis result and the elapsed time.

The specifying unit 68 determines whether or not the contrast medium permeates sufficiently, particularly, whether or not the contrast medium permeates sufficiently a region of interest, by performing the image analysis about the contrast amount on the difference image. As an example, in the present embodiment, in a case in which a brightness value of the region of interest is higher than that of the surroundings in the difference image, specifically, in a case in which a contrast of the region of interest is equal to or greater than a threshold value, it is assumed that the contrast medium has sufficiently permeated the region of interest. In the present embodiment, as long as the high-energy image is captured in a state in which the contrast medium has sufficiently permeated the region of interest, the re-capturing may not be performed. Therefore, in a case in which the contrast of the region of interest in the difference image is equal to or greater than the threshold value, the specifying unit 68 specifies that the re-capturing is not to be performed. It should be noted that, the threshold value for determining whether or not the contrast medium has sufficiently permeated the region of interest may be determined in advance according to a type of the contrast medium, a mammary gland mass of the breast, a type of an object of interest, and the like. In addition, the threshold value may be able to be set by the user.

On the other hand, the ease of permeation of the contrast medium tends to differ according to the type of the contrast medium, a blood flow of the breast, the mammary gland mass of the breast, a height of the examinee, a weight of the examinee, the type of the object of interest, and the like. It can be said that as the speed at which the contrast medium permeates is faster, the contrast medium more easily permeates.

For example, even in a contrast medium using iodine, the speed at which the contrast medium permeates the region of interest tends to differ according to an iodine content in the contrast medium. As described above, the ease of permeation of the contrast medium tends to differ according to the type of the contrast medium. It should be noted that, regarding the "type of the contrast medium", it is said that the type of the contrast medium differs in a case in which at least one of a contrast component, such as iodine contained in the contrast medium, or a content of the contrast component in the contrast medium differs.

In addition, for example, since the contrast medium flows through a blood vessel while being mixed with blood, a permeation speed of the contrast medium tends to increase as the blood flow rate is increased. As described above, the ease of permeation of the contrast medium tends to differ according to the blood flow of the breast. It should be noted that the blood flow of the examinee may be applied instead of the blood flow of the breast itself.

In addition, for example, as the mammary gland mass of the breast is larger, the contrast medium tends to more easily permeate. Therefore, the contrast medium tends to more easily permeate in a case in which the breast is a so-called dense breast. On the other hand, since a probability of hiding in the background mammary gland is higher as the mammary gland mass is larger, it may be preferable to perform imaging early, regardless of whether or not the contrast medium has sufficiently permeated. In addition, for example, as the subject tends to be more obese, the contrast medium tends to be more hard to permeate.

In addition, for example, the contrast medium tends to more easily permeate in a lesion, such as a tumor, than the mammary gland. Also, as the lesion is more malignant, the contrast medium tends to permeate faster and the contrast medium tends to be washed out faster. However, in some cancers, such as mucous cancer, the speed at which the contrast medium permeates tends to be relatively slow. Therefore, the ease of permeation of the contrast medium tends to differ according to the type of the region of interest or whether or not the lesion is malignant.

In a case in which the contrast of the region of interest in the difference image is smaller than the threshold value, there are a case in which the contrast medium has not yet sufficiently permeated, a case in which washing out of the contrast medium that has permeated is started, and a case in which a permeation amount of the contrast medium is small for some reason. Therefore, the specifying unit 68 according to the present embodiment specifies that the re-capturing of the high-energy image is to be performed in a case in which the contrast of the region of interest in the difference image is smaller than the threshold value and the elapsed time is smaller than the threshold value. That is, the specifying unit 68 specifies that the re-capturing of the high-energy image is to be performed in a case in which the contrast medium has not yet sufficiently permeated the region of interest. On the other hand, the specifying unit 68 according to the present embodiment specifies that the re-capturing of the high-energy image is not to be performed in a case in which the contrast of the region of interest in the difference image is smaller than the threshold value and the elapsed time is equal to or longer than the threshold value. That is, the specifying unit 68 specifies that the re-capturing of the high-energy image is not to be performed in a case in which washing out of the contrast medium that has permeated the region of interest is started, or in a case in which the permeation amount of the contrast medium is small for some reason.

It should be noted that, as described above, since the permeation speed of the contrast medium differs according to the type of the contrast medium, the blood flow of the breast, the mammary gland mass of the breast, the height of the examinee, the weight of the examinee (weight of the subject), and the type of the object of interest, it is preferable that the threshold value time is determined according to at least one of the type of the contrast medium, the blood flow of the breast, the mammary gland mass of the breast, the height of the examinee, the weight of the examinee (weight of the subject), or the type of the object of interest.

In addition, in a case in which the re-capturing of the high-energy image is performed, the specifying unit 68 derives a timing for performing the re-capturing. As an example, in the present embodiment, a timing at which the elapsed time reaches a threshold value time is used as the timing performing the re-capturing of the high-energy image. Therefore, the specifying unit 68 derives the timing performing the re-capturing of the high-energy image based on the threshold value time and the elapsed time. In a case in which the re-capturing of the high-energy image is performed, the specifying unit 68 outputs information indicating the timing performing the re-capturing to the control unit 60.

In addition, the console 12 according to the present embodiment comprises a display control unit 69. As an example, in the console 12 according to the present embodiment, the CPU 50A of the control unit 50 functions as the display control unit 69 by the CPU 50A executing the difference image display program 51B stored in the ROM 50B.

The display control unit 69 has a function of displaying the difference image generated by the generation unit 66 on the display unit 58. In addition, the display control unit 69 according to the present embodiment derives the contrast amount information about the contrast amount from the difference image. Examples of the contrast amount information include a numerical value indicating the contrast amount. For example, in a case of the numerical value indicating the contrast amount of the region of interest, the display control unit 69 first specifies the region of interest from the difference image. It should be noted that a method by which the display control unit 69 specifies the region of interest from the difference image is not particularly limited. For example, the region of interest may be specified from the difference image by receiving information about the region of interest input by the user. Specifically, at least one image of the difference image, the low-energy image, or the high-energy image may be displayed on the display unit 58, and a region designated by the user operating the operation unit 56 on the display image may be received as the information about the region of interest. In addition, for example, the display control unit 69 may specify the region of interest by applying computer aided diagnosis (CAD) to the difference image.

A pixel value of a pixel in the difference image corresponds to the contrast amount. Therefore, it is possible to derive the contrast amount from the pixel value of the difference image. The display control unit 69 derives the contrast amount based on the pixel value of the pixel of the image corresponding to the specified region of interest. It should be noted that the display control unit 69 may derive any of a total value, an average value, a median value, a maximum value, and the like of the contrast amount of the entire region of interest, and which value to derive may be determined in advance, or may be able to be designated by the user. In addition, the display control unit 69 may derive the numerical value indicating the contrast amount regardless of the region of interest. For example, the display control unit 69 may derive the numerical value indicating the contrast amount of the position or the region indicated by the user for the difference image or the like. In addition, for example, the display control unit 69 may derive the numerical value indicating the contrast amount of the region outside the specified region of interest.

Next, an action of the console 12 in the contrast imaging by the radiography system 1 according to the present embodiment will be described with reference to the drawings.

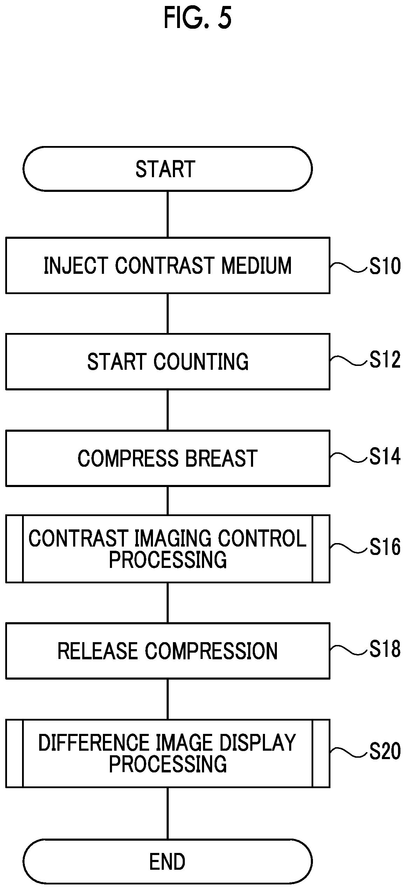

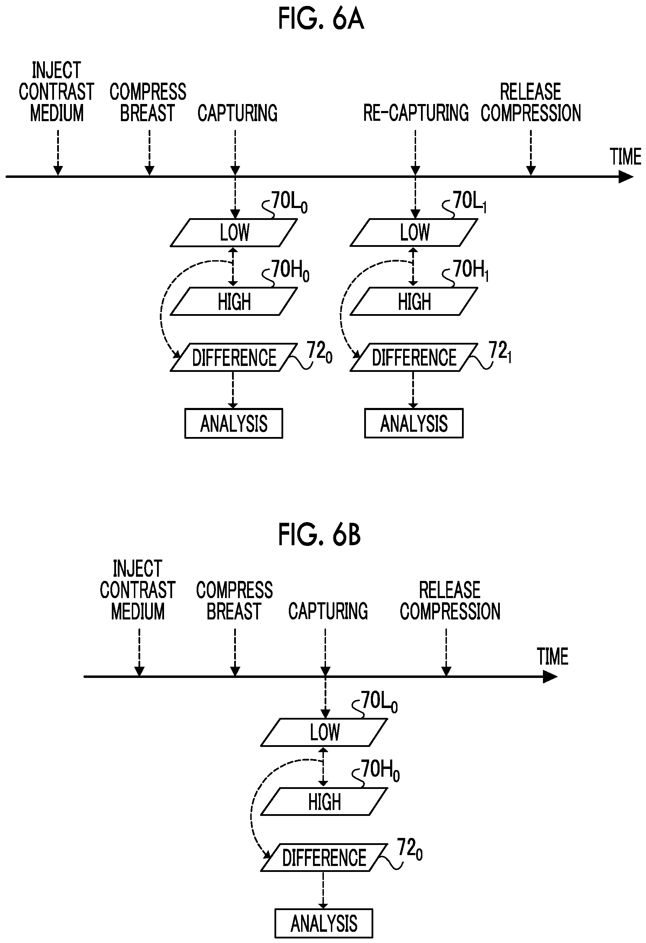

Fig. 5 shows a flowchart showing an example of a flow of the contrast imaging by the radiography system 1 according to the present embodiment. In addition, Fig. 6A shows an example of a time chart of the contrast imaging in a case in which the re-capturing is performed. In addition, Fig. 6B shows an example of a time chart of the contrast imaging in a case in which the re-capturing is not performed. It should be noted that, in the present embodiment, in a case in which a captured low-energy image 70L₀ and a re-captured low-energy image 70L₁ are collectively referred to without distinction, the captured low-energy image 70L₀ and the re-captured low-energy image 70L₁ may be referred to as a low-energy image 70L. Similarly, in the present embodiment, in a case in which a first captured high-energy image 70H₀ and a re-captured high-energy image 70H₁ are collectively referred to without distinction, the first captured high-energy image 70H₀ and the re-captured high-energy image 70H₁ may be referred to as a high-energy image 70H. In addition, in the present embodiment, in a case in which a difference image 72₀ showing a difference between the low-energy image 70L₀ and the high-energy image 70H₀, which are first captured, and a difference image 72₁ showing a difference between the low-energy image 70L₁ and the high-energy image 70H₁, which are re-captured, are collectively referred to without distinction, the difference image 72₀ and the difference image 72₁ may be referred to as a difference image 72.

In a case in which the contrast imaging is performed, first, the user injects the contrast medium into the breast, which is the subject, as shown in step S10 of Fig. 5. As shown in next step S12, the user operates the operation unit 56 to start counting by the timer 57. Next, as shown in step S14, the user positions the breast of the examinee on the imaging table 30 of the mammography apparatus 10 and compresses the breast with the compression plate 40.

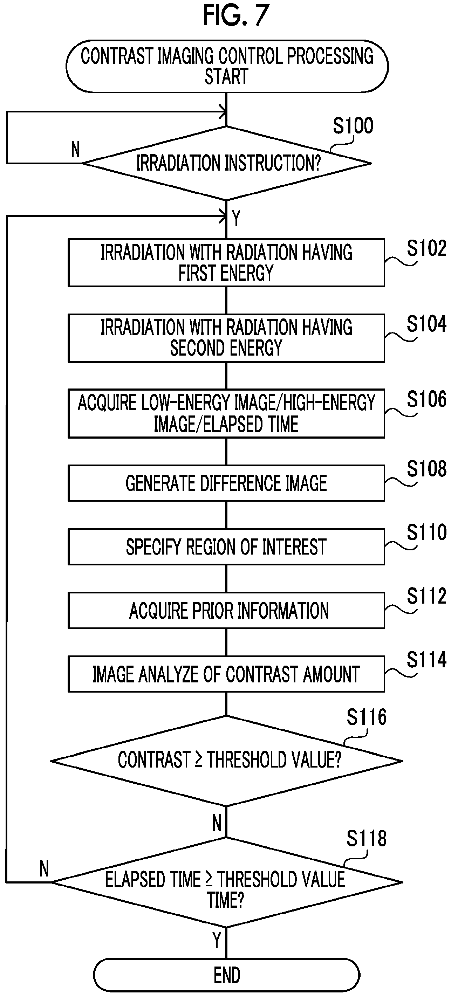

Next, in step S16, the contrast imaging control processing shown in Fig. 7 of capturing the low-energy image 70L and the high-energy image 70H by the mammography apparatus 10 is performed by the console 12. In the console 12 according to the present embodiment, as an example, the CPU 50A of the control unit 50 executes the contrast imaging control processing program 51A stored in the ROM 50B, thereby executing the contrast imaging control processing shown in Fig. 7 as an example. Fig. 7 shows a flowchart showing an example of a flow of the contrast imaging control processing executed in the console 12 according to the present embodiment.

In step S100 of Fig. 7, the control unit 60 determines whether or not the irradiation instruction of the radiation R is received. A negative determination is made in the determination in step S100 until the irradiation instruction is received. On the other hand, in a case in which the irradiation instruction is received, a positive determination is made in the determination in step S100, and the processing proceeds to step S102.

In step S102, the control unit 60 outputs the instruction to perform the irradiation with the radiation R having the first energy to the mammography apparatus 10. In the mammography apparatus 10, the control unit 20 emits the radiation R having the first energy from the radiation source 37R toward the breast based on the instruction input from the console 12, and the low-energy image 70L is captured by the radiation detector 28.

In step S104, the control unit 60 outputs an instruction to perform the irradiation with the radiation R having the second energy to the mammography apparatus 10, and then proceeds to step S106. In the mammography apparatus 10, the control unit 20 emits the radiation R having the second energy from the radiation source 37R toward the breast based on the instruction input from the console 12, and the high-energy image 70H is captured by the radiation detector 28.

It should be noted that the order in which the low-energy image 70L and the high-energy image 70H are captured is not limited to the present embodiment, and the high-energy image 70H may be captured before the low-energy image 70L. That is, the order of the processing of step S102 and the processing of step S104 may be switched.

In next step S106, the first acquisition unit 62 acquires the elapsed time, as described above. In addition, the second acquisition unit 64 acquires the low-energy image 70L and the high-energy image 70H, as described above.

In next step S108, as described above, the generation unit 66 generates the difference image 72 from the low-energy image 70L and the high-energy image 70H acquired in step S106. In the examples shown in Figs. 6A and 6B, the generation unit 66 generates the difference image 72₀ showing the difference between the low-energy image 70L₀ and the high-energy image 70H₀.

In next step S110, the specifying unit 68 specifies the region of interest in the difference image 72. As an example, the specifying unit 68 according to the present embodiment specifies the region of interest by applying the CAD to the difference image 72 generated in step S108.

In next step S112, the specifying unit 68 acquires prior information. The prior information is information for specifying the threshold value time, and is information obtained before the current contrast imaging. As described above, as an example, the threshold value time according to the present embodiment is determined according to at least one of the type of the contrast medium, the blood flow of the breast, the mammary gland mass of the breast, the height of the examinee, the weight of the examinee, or the type of the object of interest. A method by which the specifying unit 68 acquires the prior information is not particularly limited.

For example, in a case in which an image captured in advance for the breast that is currently undergoing the contrast imaging is present, the specifying unit 68 may acquire the prior information from the image captured in advance. Examples of the image captured in advance include a radiation image, an ultrasound image, a computed tomography (CT) image, and a magnetic resonance imaging (MRI) image obtained by performing the general imaging on the breast that is currently undergoing the contrast imaging. The specifying unit 68 applies the CAD to at least one of these images to acquire the mammary gland mass of the breast, the presence or absence of the region of interest, the degree of malignancy of the lesion, and the like as the type of the object of interest. Alternatively, in a case in which an interpretation result of a doctor or the like is added to these images, the specifying unit 68 acquires the interpretation result as the prior information.

In addition, for example, the specifying unit 68 may acquire the prior information, such as the type of the contrast medium, the blood flow of the breast, the height of the examinee, the weight of the examinee, and the type of the object of interest, from an imaging menu, an imaging order, an electronic medical record, and the like. In addition, for example, the specifying unit 68 may acquire the prior information input by the user using the operation unit 56.

It should be noted that since there is a case in which the specifying unit 68 acquires a plurality of types of prior information, priorities may be set in advance for the plurality of types of prior information described above, or priorities may be able to be set by the user. For example, the specifying unit 68 may adopt the latest prior information at the present time. In addition, for example, in a case in which a malignant lesion is specified as the object of interest, the specifying unit 68 may adopt the prior information about the specified malignant lesion regardless of a point in time of the prior information in which the malignant lesion is specified.

It should be noted that, in a case in which the prior information that can be acquired by the specifying unit 68 is not present, the specifying unit 68 specifies that the prior information cannot be acquired.

In next step S114, as described above, the specifying unit 68 performs the image analysis about the contrast amount on the difference image 72. In the present embodiment, the contrast of the region of interest in the difference image 72 is derived as the analysis result, as described above.

In next step S116, as described above, the specifying unit 68 determines whether or not the contrast derived in step S114 is equal to or greater than the threshold value (contrast ≥ threshold value). In a case in which the contrast is not equal to or greater than the threshold value, in other words, in a case in which the contrast is smaller than the threshold value, a negative determination is made in the determination in step S116, and the processing proceeds to step S118.

In step S118, the specifying unit 68 determines whether or not the elapsed time is equal to or longer than the threshold value time (elapsed time ≥ threshold value time), as described above. The threshold value time used in the present processing is specified according to the prior information acquired in step S112. As an example, in the present embodiment, correspondence relationship information indicating a correspondence relationship between the prior information and the threshold value time is stored in advance in the storage unit 52. The specifying unit 68 specifies the threshold value time corresponding to the prior information acquired in step S112 with reference to the correspondence relationship information stored in the storage unit 52. In a case in which the elapsed time is not equal to or longer than the threshold value time, in other words, in a case in which the elapsed time is shorter than the threshold value time, it can be assumed that the contrast medium has not yet sufficiently permeated, as described above. Therefore, a negative determination is made in the determination in step S118, and the specifying unit 68 specifies that the re-capturing of the high-energy image 70H is to be performed, returns to step S102, and repeats the processing of steps S102 to S116. As a result, for example, the re-capturing is performed as shown in Fig. 6A.

On the other hand, in a case in which the elapsed time is equal to or longer than the threshold value time, as described above, washing out of the contrast medium is started or the permeation amount of the contrast medium is small for some reason, so that the specifying unit 68 specifies that the re-capturing of the high-energy image 70H is not to be performed, and the contrast imaging control processing shown in Fig. 7 ends.

In addition, in a case in which the contrast is equal to or greater than the threshold value in step S116, it can be assumed that the contrast medium has sufficiently permeated the contrast medium, as described above. Therefore, a positive determination is made in the determination in step S116, and the contrast imaging control processing shown in Fig. 7 ends.

In this way, in a case in which the contrast imaging control processing shown in Fig. 7 ends, the processing of step S16 shown in Fig. 5 ends. In a case in which the processing of step S16 ends, the contrast imaging ends. It should be noted that the control unit 60 may notify the user that the contrast imaging ends.

Therefore, in next step S18, the compression of the breast is released. Specifically, the control unit 60 outputs an instruction to the mammography apparatus 10 to move the compression plate 40 in a direction away from the imaging table 30. In the mammography apparatus 10, the control unit 50 moves the compression plate 40 in the direction away from the imaging table 30 based on the input instruction. As a result, the compression of the breast is released. It should be noted that the release of the breast compression may be performed according to the instruction of the user, or may be performed automatically according to the end of the contrast imaging.

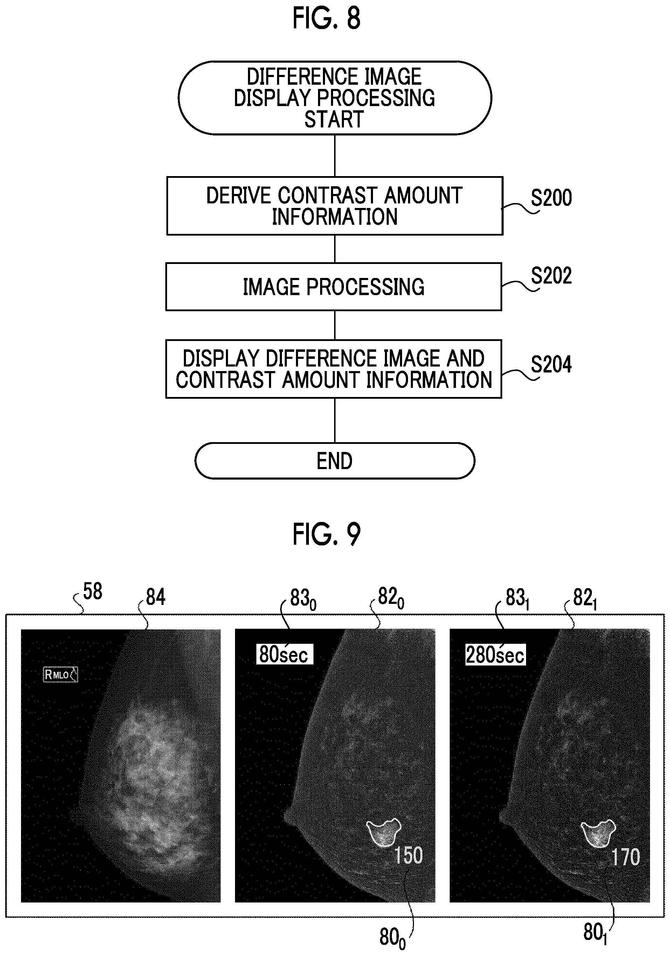

In next step S20, the difference image display processing shown in Fig. 8 is performed by the console 12. In the console 12 according to the present embodiment, as an example, the CPU 50A of the control unit 50 executes the difference image display program 51B stored in the ROM 50B, thereby executing the difference image display processing shown in Fig. 8 as an example. Fig. 8 shows a flowchart showing an example of a flow of the difference image display processing executed in the console 12 according to the present embodiment.

In step S200, the display control unit 69 derives the contrast amount information, as described above. Specifically, the contrast amount information is derived from the difference image 72 generated in step S108 of the contrast imaging control processing shown in Fig. 7 executed in step S16.

In next step S202, the display control unit 69 performs the image processing of enhancing the difference image 72. Examples of the image processing performed by the display control unit 69 include gradation enhancement processing and frequency enhancement processing. The display control unit 69 according to the present embodiment performs an analysis, such as a histogram analysis, on the difference image 72 generated by the generation unit 66, specifies which image processing, the gradation enhancement processing or the frequency enhancement processing, to perform, and specifies a degree of enhancement. The display control unit 69 performs the image processing on the difference image 72 according to the specified image processing and degree of enhancement, and generates a difference image 82 after the image processing (hereinafter, simply referred to as a "difference image 82").

It should be noted that, in order to facilitate the comparison between the difference image 72₀ and the difference image 72₁, it is preferable that the display control unit 69 performs the same processing as the image processing performed on the difference image 72₀ and the image processing performed on the difference image 72₁. In addition, it is preferable that the degree of enhancement in the image processing performed on the difference image 72₀ by the display control unit 69 and the degree of enhancement in the image processing performed on the difference image 72₁ by the display control unit 69 are the same.

In next step S204, the display control unit 69 performs control of displaying, on the display unit 58, the contrast amount information derived in step S200 and the difference image 82 after the image processing in step S202. In a case in which the processing of step S204 ends, the difference image generation processing shown in Fig. 8 ends.

Fig. 9 shows an example of a state in which the difference image 82 and the contrast amount information 80 are displayed on the display unit 58. As shown in Fig. 9, the display control unit 69 according to the present embodiment displays a difference image 82₀ obtained by performing the image processing on the difference image 72₀ and a difference image 82₁ obtained by performing the image processing on the difference image 72₁ obtained by re-capturing side by side on the display unit 58. In addition, in the example shown in Fig. 9, the display control unit 69 displays contrast amount information 80₀ of the region of interest and elapsed time information 83₀ indicating the elapsed time corresponding to the difference image 82₀ in a manner being superimposed on the difference image 82₀. In addition, the display control unit 69 displays contrast amount information 80₁ of the region of interest and elapsed time information 83₁ indicating the elapsed time corresponding to the difference image 82₁ in a manner being superimposed on the difference image 82₁.

In addition, as shown in Fig. 9, the display control unit 69 also displays a radiation image 84 on the display unit 58 as a comparative example in a case in which there is the radiation image 84 captured by the general imaging for the breast which is the subject, in other words, the radiation image 84 captured in a state in which the contrast medium has not been injected. It should be noted that, as described above, since almost no contrast medium is reflected and the body tissue, such as the mammary gland, is clearly reflected in the low-energy image, the low-energy image 70L may be displayed on the display unit 58 instead of the radiation image 84.

In addition, in the example shown in Fig. 9, the form has been described in which the difference image 82₀ and the difference image 82₁ are displayed side by side. However, a form may be adopted in which any one of the difference image 82₀ or the difference image 82₁ is displayed and the difference image to be displayed is switched according to the instruction of the user.

In this way, in a case in which the difference image display processing shown in Fig. 8 ends, the difference image display processing in step S20 shown in Fig. 5 ends. As a result, the series of processing related to the contrast imaging in the radiography system 1 according to the present embodiment ends. It should be noted that a form may be adopted in which the low-energy image 70L and the plurality of high-energy images 70H, which are captured by the mammography apparatus 10 according to the present embodiment, the elapsed time information 83, the plurality of difference images 72, the difference image 82 after the image processing, and the contrast amount information 80, which are generated by the console 12, and the like are stored in the storage unit 52 of the console 12, picture archiving and communication systems (PACS), or the like.

In addition, in the form described above, the form has been described in which the difference image display processing is continuously performed after the processing of S18 of Fig. 5 ends. However, the timing for performing the difference image display processing, that is, the timing for displaying the difference image 82 is not limited to the present form. For example, a form may be adopted in which the timing for displaying the difference image 82 is a timing according to the user's desire after the contrast imaging.

As described above, the console 12 according to the form described above comprises the CPU 50A as at least one processor. The CPU 50A acquires the elapsed time from the injection of the contrast medium into the breast for which the radiation image is captured by the mammography apparatus 10. In addition, the CPU 50A acquires the low-energy image captured by the mammography apparatus 10 by emitting the radiation R having the first energy to the breast into which the contrast medium has been injected, and the high-energy image captured by the mammography apparatus 10 by emitting the radiation R having the second energy higher than the first energy to the breast into which the contrast medium has been injected. In addition, the CPU 50A generates the difference image showing the difference between the low-energy image and the high-energy image. In addition, the CPU 50A specifies whether or not the re-capturing of the high-energy image of the breast into which the contrast medium has been injected is to be performed, based on the analysis result about the contrast amount, which is performed on the difference image, and the elapsed time.

The degree of permeation of the contrast medium into the breast is changed depending on various conditions, such as the mammary gland mass of the breast or the type of the object of interest. Therefore, it may be difficult to understand the imaging timing. In addition, it may be difficult to determine whether or not it can be assumed that the contrast medium has sufficiently permeated even with reference to the captured difference image. In response to such a case, in the present embodiment, it is specified whether or not the re-capturing of the high-energy image is to be performed, based on the analysis result about the contrast amount, which is performed on the difference image showing the difference between the low-energy image and the high-energy image, and the elapsed time. Therefore, according to the present embodiment, it is possible to capture the high-energy image at an appropriate timing in the contrast imaging.

In addition, according to the present embodiment, since the number of times of re-capturing can be set to an appropriate number of times, an exposure dose of the subject can be suppressed.

It should be noted that, in the form described above, for the re-capturing, the form has been described in which both the low-energy image and the high-energy image are re-captured, but the re-capturing is not limited to the present form, and a form need only be adopted in which the re-capturing of at least the high-energy image is performed. That is, the low-energy image may not be re-captured. As described above, in the low-energy image, the mammary gland structure is clearly reflected, but almost no contrast medium is reflected. Therefore, the low-energy image tends to be the same image regardless of the permeation of the contrast medium due to the lapse of time. Therefore, the low-energy image may not be re-captured more frequently than the high-energy image.

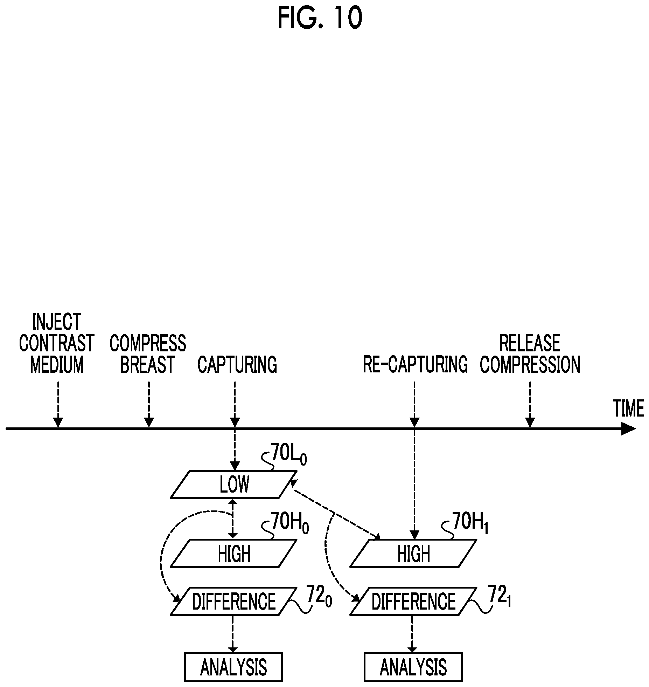

Fig. 10 shows an example of a time chart in a case in which the re-capturing of only the high-energy image is performed. In the example shown in Fig. 10, after the compression of the breast, the low-energy image 70L₀ and the high-energy image 70H₀ are first captured, and the generation unit 66 generates the difference image 72₀. In a case in which the specifying unit 68 specifies that the re-capturing is to be performed based on the analysis result of the image analysis of the difference image 72, the control unit 60 re-captures only the high-energy image 70H₁. Then, the generation unit 66 generates the difference image 72₁ between the low-energy image 70L₀ and the high-energy image 70H₁.

By reducing the number of times of capturing of the low-energy image by re-capturing only the high-energy image in this way, it is possible to reduce the exposure dose of the examinee. On the other hand, since there is a case in which the body movement, such as the movement of the mammary gland structure, occurs in the breast, it is preferable to re-capture the low-energy image in consideration of the body movement.

It should be noted that, in the form described above, the form has been described in which the re-capturing is performed until a positive determination is made in step S116 of the contrast imaging control processing (see Fig. 7) or a positive determination is made in step S118. That is, the form has been described in which the re-capturing is performed a plurality of times until the condition described above is satisfied. However, the number of times of re-capturing is not limited, and for example, an upper limit number of times may be set, or the re-capturing may be performed only once.

It should be noted that, in the form described above, the form has been described in which the re-capturing of the high-energy image and the low-energy image is automatically performed, but a form may be adopted in which the re-capturing may be performed according to the instruction of the user. For example, a form may be adopted in which, in a case in which the specifying unit 68 specifies that the re-capturing is to be performed, the timing for performing the re-capturing is notified to the user, and the control unit 60 performs the re-capturing according to the instruction for the re-capturing by the user in response to the notification.

In addition, in the present embodiment, the form has been described in which the console 12 comprises the timer 57 and the console 12 itself counts the elapsed time, but a form may be adopted in which the console 12 does not comprise the timer 57 and counts the elapsed time by an external apparatus. For example, a form may be adopted in which the user measures the elapsed time by a measuring instrument, such as a stopwatch and inputs the elapsed time to the console 12 using the operation unit 56, and the first acquisition unit 62 acquires the input elapsed time.

In addition, in the form described above, the form has been described in which the timing for the threshold value time or performing the re-capturing is specified based on the prior information, but the timing for first capturing the low-energy image and the high-energy image after the injection of the contrast medium may be derived based on the prior information. It should be noted that, in this case, the timing for first capturing the low-energy image and the high-energy image and the timings for the threshold value time and performing the re-capturing are set as different timings.

In addition, in the form described above, the form has been described in which the breast is applied as an example of the subject according to the present disclosure, and the mammography apparatus 10 is applied as an example of the radiography apparatus according to the present disclosure, but the subject is not limited to the breast, and the radiography apparatus is not limited to the mammography apparatus. For example, the subject may be a chest, an abdomen, or the like, and a form may be adopted in which a radiography apparatus other than the mammography apparatus is applied as the radiography apparatus.

In addition, in the form described above, the form has been described in which the console 12 is an example of the control apparatus according to the present disclosure, but an apparatus other than the console 12 may have the function of the control apparatus according to the present disclosure. In other words, some or all of the functions of the control unit 60, the first acquisition unit 62, the second acquisition unit 64, the generation unit 66, the specifying unit 68, and the display control unit 69 may be provided in an apparatus other than the console 12, for example, the mammography apparatus 10 or an external apparatus.

In addition, in the form described above, various processors shown below can be used as the hardware structure of processing units that execute various pieces of processing, such as the control unit 60, the first acquisition unit 62, the second acquisition unit 64, the generation unit 66, the specifying unit 68, and the display control unit 69. As described above, the various processors include, in addition to the CPU which is a general-purpose processor which executes software (program) and functions as various processing units, a programmable logic device (PLD) which is a processor of which a circuit configuration can be changed after manufacture, such as a field programmable gate array (FPGA), and a dedicated electric circuit which is a processor having a circuit configuration which is designed for exclusive use in order to execute specific processing, such as an application specific integrated circuit (ASIC).

One processing unit may be composed of one of the various processors or may be composed of a combination of two or more processors of the same type or different types (for example, a combination of a plurality of FPGAs or a combination of a CPU and an FPGA). In addition, a plurality of processing units may be composed of one processor.

A first example of the configuration in which the plurality of processing units are composed of one processor is a form in which one processor is composed of a combination of one or more CPUs and software and the processor functions as the plurality of processing units, as represented by the computer, such as a client and a server. Second, as represented by a system on chip (SoC) or the like, there is a form of using a processor that realizes the function of the entire system including the plurality of processing units by one integrated circuit (IC) chip. As described above, various processing units are composed of one or more of the various processors as the hardware structure.

Further, more specifically, an electric circuit (circuitry) obtained by combining circuit elements, such as semiconductor elements, can be used as the hardware structure of the various processors.

In addition, in each embodiment described above, the aspect has been described in which the contrast imaging control processing program 51A and the difference image display program 51B are stored (installed) in the ROM 50B in advance, but the present disclosure is not limited to this. Each of the contrast imaging control processing program 51A and the difference image display program 51B may be provided in a form being recorded in the recording medium, such as a compact disc read only memory (CD-ROM), a digital versatile disc read only memory (DVD-ROM), and a universal serial bus (USB) memory. In addition, a form may be adopted in which each of the contrast imaging control processing program 51A and the difference image display program 51B is downloaded from an external apparatus via a network.

## Claims

1. A control apparatus (50) comprising:
at least one processor (50A) that is configured to:
acquire an elapsed time from injection of a contrast medium into a subject for which a radiation image (84) is captured by a radiography apparatus (1),
acquire a low-energy image (70L₀) captured by the radiography apparatus (1) by emitting radiation having first energy to the subject into which the contrast medium has been injected, and a high-energy image (70H₀) captured by the radiography apparatus (1) by emitting radiation having second energy higher than the first energy to the subject into which the contrast medium has been injected,
generate a difference image (72₀) showing a difference between the low-energy image (70L₀) and the high-energy image (70H₀), and
the control apparatus (50) **characterised in that** the at least one processor (50A) is configured to:
identify whether or not to perform re-capturing of the high-energy image (70H₀) of the subject into which the contrast medium has been injected, based on an analysis result about a contrast amount, which is performed on the difference image (72₀), and the elapsed time.

2. The control apparatus (50) according to claim 1,
wherein the at least one processor (50A) is configured to, in a case in which the at least one processor (50A) identifies that the re-capturing is to be performed, derive a timing for performing the re-capturing based on the analysis result and the elapsed time.

3. The control apparatus (50) according to claim 2,
wherein the at least one processor (50A) is configured to perform control of causing the radiography apparatus (1) to re-capture the high-energy image (70H₀) at the derived timing for performing the re-capturing.

4. The control apparatus (50) according to any one of claims 1 to 3,
wherein the at least one processor (50A) is configured to, in a case in which a contrast of a region of interest in the difference image (72₀) is equal to or greater than a predetermined threshold value, identify that the re-capturing is not to be performed regardless of the elapsed time.

5. The control apparatus (50) according to any one of claims 1 to 4,
wherein the at least one processor (50A) is configured to, in a case in which a contrast of a region of interest in the difference image (72₀) is smaller than a predetermined threshold value and the elapsed time is equal to or longer than a threshold value time, identify that the re-capturing is not to be performed.

6. The control apparatus (50) according to any one of claims 1 to 4,
wherein the at least one processor (50A) is configured to, in a case in which a contrast of a region of interest in the difference image (72₀) is smaller than a predetermined threshold value and the elapsed time is shorter than a threshold value time, identify that the re-capturing is to be performed.

7. The control apparatus (50) according to claim 5 or 6,
wherein the threshold value time is a time according to at least one of a type of the contrast medium, a blood flow of the subject, a height of the subject, a weight of the subject, or a type of an object of interest.

8. The control apparatus (50) according to claim 5 or 6,
wherein the subject is a breast, and
the threshold value time is a time according to a mammary gland mass of the breast.

9. The control apparatus (50) according to any one of claims 1 to 8,
wherein, in a case in which the at least one processor (50A) identifies that the re-capturing is to be performed, the at least one processor (50A) is configured to:
acquire the high-energy image (70H₁) re-captured by the radiography apparatus (1), and
generate a re-captured difference image (72₁) showing a difference between the low-energy image (70L₀) and the re-captured high-energy image (70H₁).

10. The control apparatus (50) according to any one of claims 1 to 8,
wherein, in a case in which the at least one processor (50A) identifies that the re-capturing is to be performed, the at least one processor (50A) is configured to:
also identify that re-capturing of the low-energy image (70L₀) is to be performed, and
generate a re-captured difference image (72₁) showing a difference between the re-captured low-energy image (70L₁) and the re-captured high-energy image (70H₁).

11. The control apparatus (50) according to claim 9 or 10,
wherein the at least one processor (50A) is configured to:
perform the same image processing of enhancing a region of interest on the difference image (72₀) and the re-captured difference image (72₁), and
display the difference image (82₀) and the re-captured difference image (82₁) which have been subjected to the image processing.

12. The control apparatus (50) according to any one of claims 9 to 11,
wherein the at least one processor (50A) is further configured to display contrast amount information about a contrast amount of the same region of each of the difference image (72₀) and the re-captured difference image (72₁).

13. The control apparatus (50) according to any one of claims 1 to 12,
wherein the subject is a breast, and
the radiography apparatus (1) is a mammography apparatus (10).

14. A non-transitory storage medium storing a program causing a computer to execute a control processing, the control processing comprising:
acquiring an elapsed time from injection of a contrast medium into a subject for which a radiation image is captured by a radiography apparatus (1);
acquiring a low-energy image (70L₀) captured by the radiography apparatus (1) by emitting radiation having first energy to the subject into which the contrast medium has been injected, and a high-energy image (70H₀) captured by the radiography apparatus (1) by emitting radiation having second energy higher than the first energy to the subject into which the contrast medium has been injected;
generating a difference image (72₀) showing a difference between the low-energy image (70L₀) and the high-energy image (70H₀); and
**characterised in that** the control processing further comprises:
identifying whether or not to perform re-capturing of the high-energy image (70H₀) of the subject into which the contrast medium has been injected, based on an analysis result about a contrast amount, which is performed on the difference image (72₀), and the elapsed time.

## Patentansprüche

1. Steuervorrichtung (50), umfassend:
mindestens einen Prozessor (50A), der so konfiguriert ist, dass er:
eine verstrichene Zeit seit Injektion eines Kontrastmittels in ein Objekt, für das ein Strahlungsbild (84) von einer Röntgenvorrichtung (1) aufgenommen wurde, erfasst,
ein Niedrigenergiebild (70L₀), das von der Röntgenvorrichtung (1) aufgenommen wurde, durch Emittieren von Strahlung mit erster Energie auf das Objekt, in das das Kontrastmittel injiziert wurde, und ein Hochenergiebild (70H₀), das von der Röntgenvorrichtung (1) aufgenommen wurde, durch Emittieren von Strahlung mit zweiter Energie, die höher als die erste Energie ist, auf das Objekt, in das das Kontrastmittel injiziert wurde, erfasst,
ein Differenzbild (72₀), das einen Unterschied zwischen dem Niedrigenergiebild (70L₀) und dem Hochenergiebild (70H₀) zeigt, erzeugt, und
die Steuervorrichtung (50), **dadurch gekennzeichnet, dass** der mindestens eine Prozessor (50A) so konfiguriert ist, dass er:
auf der Grundlage eines Analyseergebnisses über eine Kontrastmenge, die an dem Differenzbild (72₀) durchgeführt wurde, und der verstrichenen Zeit feststellt, ob erneute Aufnahme des Hochenergiebildes (70H₀) des Objekts, in das das Kontrastmittel injiziert wurde, durchgeführt werden soll oder nicht.

2. Steuervorrichtung (50) nach Anspruch 1,
wobei der mindestens eine Prozessor (50A) so konfiguriert ist, dass er in einem Fall, in dem der mindestens eine Prozessor (50A) feststellt, dass die erneute Aufnahme durchgeführt werden soll, einen Zeitpunkt zum Durchführen der erneuten Aufnahme auf der Grundlage des Analyseergebnisses und der verstrichenen Zeit ableitet.

3. Steuervorrichtung (50) nach Anspruch 2,
wobei der mindestens eine Prozessor (50A) so konfiguriert ist, dass er Steuerung zum Veranlassen der Röntgenvorrichtung (1), das Hochenergiebild (70H₀) zu dem abgeleiteten Zeitpunkt zum Durchführen der erneuten Aufnahme erneut aufzunehmen, durchführt.

4. Steuervorrichtung (50) nach einem der Ansprüche 1 bis 3,
wobei der mindestens eine Prozessor (50A) so konfiguriert ist, dass er in einem Fall, in dem ein Kontrast eines Bereichs von Interesse in dem Differenzbild (72₀) gleich oder größer als ein vorbestimmter Schwellenwert ist, feststellt, dass die erneute Aufnahme unabhängig von der verstrichenen Zeit nicht durchgeführt werden soll.

5. Steuervorrichtung (50) nach einem der Ansprüche 1 bis 4,
wobei der mindestens eine Prozessor (50A) so konfiguriert ist, dass er in einem Fall, in dem ein Kontrast eines Bereichs von Interesse in dem Differenzbild (72₀) kleiner als ein vorbestimmter Schwellenwert ist und die verstrichene Zeit gleich oder länger als eine Schwellenwertzeit ist, feststellt, dass die erneute Aufnahme nicht durchgeführt werden soll.

6. Steuervorrichtung (50) nach einem der Ansprüche 1 bis 4,
wobei der mindestens eine Prozessor (50A) so konfiguriert ist, dass er in einem Fall, in dem ein Kontrast eines Bereichs von Interesse in dem Differenzbild (72₀) kleiner als ein vorbestimmter Schwellenwert ist und die verstrichene Zeit kürzer als eine Schwellenwertzeit ist, feststellt, dass die erneute Aufnahme durchgeführt werden soll.

7. Steuervorrichtung (50) nach Anspruch 5 oder 6,
wobei die Schwellenwertzeit eine Zeit gemäß mindestens einem von einem Typ des Kontrastmittels, einem Blutfluss des Objekts, einer Körpergröße des Objekts, einem Gewicht des Objekts oder einem Typ eines Objekts von Interesse ist.

8. Steuervorrichtung (50) nach Anspruch 5 oder 6,
wobei das Objekt eine Brust ist, und
die Schwellenwertzeit eine Zeit gemäß einer Brustdrüsenmasse der Brust ist.

9. Steuervorrichtung (50) nach einem der Ansprüche 1 bis 8,
wobei der mindestens eine Prozessor (50A) in einem Fall, in dem der mindestens eine Prozessor (50A) feststellt, dass die erneute Aufnahme durchgeführt werden soll, so konfiguriert ist, dass er:
das von der Röntgenvorrichtung (1) erneut aufgenommene Hochenergiebild (70H₁) erfasst, und
ein erneut aufgenommenes Differenzbild (72₁), das einen Unterschied zwischen dem Niedrigenergiebild (70L₀) und dem erneut aufgenommenen Hochenergiebild (70H₁) zeigt, erzeugt.

10. Steuervorrichtung (50) nach einem der Ansprüche 1 bis 8,
wobei der mindestens eine Prozessor (50A) in einem Fall, in dem der mindestens eine Prozessor (50A) feststellt, dass die erneute Aufnahme durchgeführt werden soll, so konfiguriert ist, dass er:
auch feststellt, dass erneute Aufnahme des Niedrigenergiebildes (70L₀) durchgeführt werden soll, und
ein erneut aufgenommenes Differenzbild (72₁), das einen Unterschied zwischen dem erneut aufgenommenen Niedrigenergiebild (70L₁) und dem erneut aufgenommenen Hochenergiebild (70H₁) zeigt, erzeugt.

11. Steuervorrichtung (50) nach Anspruch 9 oder 10,
wobei der mindestens eine Prozessor (50A) so konfiguriert ist, dass er:
die gleiche Bildverarbeitung des Verbesserns eines Bereichs von Interesse an dem Differenzbild (72₀) und dem erneut aufgenommenen Differenzbild (72₁) durchführt, und
das Differenzbild (82₀) und das erneut aufgenommene Differenzbild (82₁), die der Bildverarbeitung unterzogen wurden, anzeigt.

12. Steuervorrichtung (50) nach einem der Ansprüche 9 bis 11,
wobei der mindestens eine Prozessor (50A) ferner so konfiguriert ist, dass er Kontrastmengeninformationen über eine Kontrastmenge derselben Region von jeweils dem Differenzbild (72₀) und dem erneut aufgenommenen Differenzbild (72₁) anzeigt.

13. Steuervorrichtung (50) nach einem der Ansprüche 1 bis 12,
wobei das Objekt eine Brust ist, und
die Röntgenvorrichtung (1) eine Mammographievorrichtung (10) ist.

14. Nicht flüchtiges Speichermedium, das ein Programm speichert, das einen Computer veranlasst, eine Steuerverarbeitung auszuführen, wobei die Steuerverarbeitung umfasst:
Erfassen einer verstrichenen Zeit seit Injektion eines Kontrastmittels in ein Objekt, für das ein Strahlungsbild von einer Röntgenvorrichtung (1) aufgenommen wurde;
Erfassen eines Niedrigenergiebildes (70L₀), das von der Röntgenvorrichtung (1) aufgenommen wurde, durch Emittieren von Strahlung mit erster Energie auf das Objekt, in das das Kontrastmittel injiziert wurde, und eines Hochenergiebildes (70H₀), das von der Röntgenvorrichtung (1) aufgenommen wurde, durch Emittieren von Strahlung mit zweiter Energie, die höher als die erste Energie ist, auf das Objekt, in das das Kontrastmittel injiziert wurde;
Erzeugen eines Differenzbildes (72₀), das einen Unterschied zwischen dem Niedrigenergiebild (70L₀) und dem Hochenergiebild (70H₀) zeigt; und
**dadurch gekennzeichnet, dass** die Steuerverarbeitung ferner umfasst:
Feststellen auf der Grundlage eines Analyseergebnisses über eine Kontrastmenge, die an dem Differenzbild (72₀) durchgeführt wurde, und der verstrichenen Zeit, ob erneute Aufnahme des Hochenergiebildes (70H₀) des Objekts, in das das Kontrastmittel injiziert wurde, durchgeführt werden soll oder nicht.

## Revendications

1. Appareil de contrôle (50) comprenant :
au moins un processeur (50A) qui est configuré pour :
acquérir un temps écoulé depuis l'injection d'un produit de contraste dans un sujet pour lequel une image de rayonnement (84) est capturée par un appareil de radiographie (1),
acquérir une image à faible énergie (70L₀) capturée par l'appareil de radiographie (1) en émettant un rayonnement ayant une première énergie vers le sujet dans lequel le produit de contraste a été injecté, et une image à haute énergie (70H₀) capturée par l'appareil de radiographie (1) en émettant un rayonnement ayant une deuxième énergie supérieure à la première énergie vers le sujet dans lequel le produit de contraste a été injecté,
générer une image de différence (72₀) montrant une différence entre l'image à faible énergie (70L₀) et l'image à haute énergie (70H₀), et
l'appareil de contrôle (50) **caractérisé en ce que** l'au moins un processeur (50A) est configuré pour :
identifier s'il faut ou non effectuer une nouvelle capture de l'image à haute énergie (70H₀) du sujet dans lequel le produit de contraste a été injecté, sur la base d'un résultat d'analyse concernant une valeur de contraste, qui est effectuée sur l'image de différence (72₀), et du temps écoulé.

2. Appareil de contrôle (50) selon la revendication 1,
dans lequel l'au moins un processeur (50A) est configuré pour, dans un cas où l'au moins un processeur (50A) identifient que la nouvelle capture doit être effectuée, dériver un moment pour effectuer la nouvelle capture sur la base du résultat de l'analyse et du temps écoulé.

3. Appareil de contrôle (50) selon la revendication 2,
dans lequel l'au moins un processeur (50A) est configuré pour effectuer une contrôle consistant à amener l'appareil de radiographie (1) à effectuer une nouvelle capture de l'image à haute énergie (70H₀) au moment dérivé pour effectuer la nouvelle capture.

4. Appareil de contrôle (50) selon l'une quelconque des revendications 1 à 3,
dans lequel l'au moins un processeur (50A) est configuré pour, dans un cas où un contraste d'une région d'intérêt dans l'image de différence (72₀) est égal ou supérieur à une valeur seuil prédéterminée, identifier que la nouvelle capture ne doit pas être effectuée quel que soit le temps écoulé.

5. Appareil de contrôle (50) selon l'une quelconque des revendications 1 à 4,
dans lequel l'au moins un processeur (50A) est configuré pour, dans un cas où un contraste d'une région d'intérêt dans l'image de différence (72₀) est inférieur à une valeur seuil prédéterminée et le temps écoulé est égal ou supérieur à une temps seuil, identifier que la nouvelle capture ne doit pas être effectuée.

6. Appareil de contrôle (50) selon l'une quelconque des revendications 1 à 4,
dans lequel l'au moins un processeur (50A) est configuré pour, dans un cas où un contraste d'une région d'intérêt dans l'image de différence (72₀) est inférieur à une valeur seuil prédéterminée et le temps écoulé est inférieur à une temps seuil, identifier que la nouvelle capture doit être effectuée.

7. Appareil de contrôle (50) selon la revendication 5 ou la revendication 6,
dans lequel le temps seuil est un temps déterminé en fonction d'au moins l'un d'un type du produit de contraste, d'un flux sanguin du sujet, d'une taille du sujet, d'un poids du sujet ou d'un type d'un objet d'intérêt.

8. Appareil de contrôle (50) selon la revendication 5 ou la revendication 6,
dans lequel le sujet est un sein, et
le temps seuil est un temps en fonction d'une masse de glande mammaire du sein.

9. Appareil de contrôle (50) selon l'une quelconque des revendications 1 à 8,
dans lequel, dans un cas où l'au moins un processeur (50A) identifie que la nouvelle capture doit être effectuée, l'au moins un processeur (50A) est configuré pour :
acquérir l'image à haute énergie (70H₁) nouvellement capturée par l'appareil de radiographie (1), et
générer une image de différence nouvellement capturée (72₁) montrant une différence entre l'image à faible énergie (70L₀) et l'image à haute énergie nouvellement capturée (70H₁).

10. Appareil de contrôle (50) selon l'une quelconque des revendications 1 à 8,
dans lequel, dans un cas où l'au moins un processeur (50A) identifie que la nouvelle capture doit être effectuée, l'au moins un processeur (50A) est configuré pour :
identifier également qu'une nouvelle capture de l'image à faible énergie (70L₀) doit être effectuée, et
générer une image de différence nouvellement capturée (72₁) montrant une différence entre l'image à faible énergie nouvellement capturée (70L₁) et l'image à haute énergie nouvellement capturée (70H₁).

11. Appareil de contrôle (50) selon la revendication 9 ou la revendication 10,
dans lequel l'au moins un processeur (50A) est configuré pour :
effectuer le même traitement d'image consistant à améliorer une région d'intérêt sur l'image de différence (72₀) et l'image de différence nouvellement capturée (72₁), et
afficher l'image de différence (82₀) et l'image de différence nouvellement capturée (82₁) qui ont été soumises au traitement d'image.

12. Appareil de contrôle (50) selon l'une quelconque des revendications 9 à 11,
dans lequel l'au moins un processeur (50A) est en outre configuré pour afficher des informations de valeur de contraste concernant une valeur de contraste de la même région de chacune de l'image de différence (72₀) et de l'image de différence nouvellement capturée (72₁).

13. Appareil de contrôle (50) selon l'une quelconque des revendications 1 à 12,
dans lequel le sujet est un sein, et
l'appareil de radiographie (1) est un appareil de mammographie (10).

14. Support de stockage non transitoire stockant un programme amenant un ordinateur à exécuter un traitement de contrôle, le traitement de contrôle comprenant :
acquérir un temps écoulé depuis l'injection d'un produit de contraste dans un sujet pour lequel une image de rayonnement est capturée par un appareil de radiographie (1) ;
acquérir une image à faible énergie (70L₀) capturée par l'appareil de radiographie (1) en émettant un rayonnement ayant une première énergie vers le sujet dans lequel le produit de contraste a été injecté, et une image à haute énergie (70H₀) capturée par l'appareil de radiographie (1) en émettant un rayonnement ayant une deuxième énergie supérieure à la première énergie vers le sujet dans lequel le produit de contraste a été injecté :
générer une image de différence (72₀) montrant une différence entre l'image à faible énergie (70L₀) et l'image à haute énergie (70H₀) ; et
**caractérisé en ce que** le traitement de contrôle comprend en outre :
identifier s'il faut ou non effectuer une nouvelle capture de l'image à haute énergie (70H₀) du sujet dans lequel le produit de contraste a été injecté, sur la base d'un résultat d'analyse concernant une valeur de contraste, qui est effectuée sur l'image de différence (72₀), et du temps écoulé.
